# EUROPEAN PATENT APPLICATION

(11) **EP 4 292 510 A1**
(43) Date of publication of application: **20.12.2023**
(21) Application number: 22179383.9
(22) Date of filing: 16.06.2022
(51) Int. Cl.: A61B 1/012, A61B 1/015, A61B 1/01, A61B 1/00, A61B 1/018

(54) **NAVIGATION CATHETER ASSEMBLY WITH ENDOSCOPIC VISION PROBE**

(71) Applicant: Lys Medical SA, 6041 Gosselies (BE)
(72) Inventor: BLANC, LOIC, 1420 BRAINE-L'ALLEUD (BE); PATERNOTTE, JEAN, 1300 LIMAL (BE); BONDUE, BENJAMIN, 1180 BRUXELLES (BE); MERTENS, BENJAMIN, 1332 GENVAL (BE)
(74) Representative: AWA Benelux

(57) **Abstract**

An assembly (100) for endoluminal navigation comprises a guiding catheter (10), an endoscopic probe (20) and a fluid delivery module (13). The guiding catheter comprises an internal lumen (12) with an outlet aperture (122) at the distal end (102). The endoscopic probe (20) comprises a flexible probe body (21) and an imaging device (22) attached to the probe body at a distal end. The probe body and the imaging device are sized to be slidingly received in the internal lumen (12). The fluid delivery module (30) arranged at the proximal end of the guiding catheter (10) comprises a first port (321) communicating with the internal lumen (12) for receiving the endoscopic probe (20), and a second port (322) in fluid communication with the internal lumen for delivering a fluid thereto. The first port (321) comprises a fluid seal (34) configured to fluid tightly seal the first inlet port (321) against the probe body (21) while the second port (322) remains in fluid communication with the internal lumen (12).

## Description

### Technical field

The present disclosure is related to catheter assemblies allowing extended navigation, particularly in smaller lumens, and endoscopic imaging beyond the reach of conventional endoscopes.

### Background art

The deep lung is a system of small to very small internal lumens (bronchi) forming a complex maze which is hard to explore. Endoscopic imaging of these structures is furthermore rendered difficult due to the presence of physiological liquids. Since pulmonologists need to access the deep lung to proceed with a biopsy during lung cancer screening, a few techniques have been developed to access the deep lung.

One such technique is fluoroscopic imaging in combination with electromagnetic navigation. The catheter comprises a trackable marker that is monitored as the catheter is moved inside the body. The position of the catheter is merged on fluoroscopic or other images obtained from external sources. A drawback of this technique is that only a virtual or indirect visualization of the target anatomical features are obtained.

US 2021/0100627 describes a flexible tubular catheter with an internal channel that is sized to receive an image capture probe. The image capture probe is extendable distally beyond a distal end of the catheter and comprises a distal portion with a stereoscopic or monoscopic camera for capturing video or ultrasound images that are provided to a tracking system. One or more external images of the patient anatomy are obtained from an external imaging device located outside the patient anatomy. The external images include images of the catheter and the imaging probe. A relative position between the imaging probe and the distal end of the catheter is determined based on the external images and/or additional sensors provided on the catheter and imaging probe, such as electromagnetic sensors. A relative orientation between the imaging probe and the distal end of the catheter is determined by aligning the catheter distal end with an anatomical landmark, and detecting the anatomical landmark in images from the imaging probe. Alternatively, sensors for determining a roll angle are provided in the catheter and the imaging probe to detect the relative orientation. By so doing, images of a target structure made by the imaging probe can be presented in a virtual anatomic model of the patient anatomy.

Drawbacks of the above system are its complexity in obtaining proper yet virtual visualization of the anatomical target location, and its high cost. Yet another drawback is that physiological fluids can render the video images taken by the imaging probe useless. For this reason, ultrasound imaging probes are preferred. However, the ultrasound images require further interpretation.

US 2020/0178775 describes a surgical instrument with a hollow guide shaft that receives both an endoscope and a dilation catheter. The endoscope can be made compact by co-axially positioning the camera, a light source and a pair of suction openings at the distal end face. In addition a plurality of irrigation openings extend radially through an annular sidewall of the endoscope. While all the camera and light source wires and the suction and irrigation channels can be embedded in a unitary endoscope body to obtain a compact and unitary structure, the diameter size of the endoscope body will necessarily increase which will degrade navigation capability of the endoscope in small cavities. Alternatively, the size of the suction and irrigation channels can be made small, but this increases pressure drop as fluid flows through the channels of the endoscope thereby limiting performance.

### Summary

There is therefore a need in the art to provide catheter systems with improved extended navigation and/or imaging capabilities over prior art catheter systems, particularly with respect to navigation and visualization in small and remote cavities, such as the deep lung. There is a need in the art to provide such catheter systems that are of reduced complexity and/or economical.

According to the present disclosure, there is therefore provided an assembly for endoluminal navigation as set out in the appended claims. Assemblies according to the present disclosure comprise a guiding catheter and an endoscopic probe. The guiding catheter comprises an elongate flexible tubular catheter body defining an internal lumen with an outlet aperture at a distal end of the guiding catheter. The endoscopic probe comprises an elongate, flexible probe body and an imaging device, particularly configured to capture visual images such as a (video) camera, attached to a distal end of the probe body. The probe body and the imaging device are sized to be slidingly received in the internal lumen.

According to a first aspect, assemblies according to the present disclosure advantageously comprise a fluid delivery module attached or attachable to a proximal end of the guiding catheter. The fluid delivery module comprises a first port configured to communicate with the internal lumen and configured to receive the endoscopic probe for deployment through the internal lumen, and a second port configured to deliver a fluid to the internal lumen. The first port comprises a fluid seal configured to fluid tightly seal the first inlet port against the probe body while the second port remains in fluid communication with the internal lumen. Advantageously, the fluid seal is configured to slidingly receive the probe body while enabling to fluid tightly seal the first inlet port. The fluid seal can be further configured to tighten a sealing member against the probe body.

One advantage of the above assembly provided with the fluid delivery module is that it allows the internal lumen of the guiding catheter to be flushed with a fluid supplied through the second inlet port while the endoscopic probe is accommodated in the internal lumen. Such a flushing enables to clean external parts of the imaging device, such as lenses, etc., which may get smudged by body fluids during endoluminal navigation. By so doing the endoscopic probe can be made of small diameter since it advantageously does not need to include any fluid supply or fluid suction channels. In addition, the combination of guiding catheter which slidingly receives the endoscopic probe allows to integrate the navigation functionalities within the guiding catheter, e.g. torque transmission capability, etc., and keep the diameter of the body of the endoscopic probe small. As a result, a very compact assembly is obtained while still being provided with full navigation and endoscopic functionalities. The assembly is further of a very simple structure, as the requirement of multiple channels, etc. can be dispensed with, and is hence more economical than prior art devices. Furthermore, due to the simple structure, some parts can be cleaned and/or sterilized easily, specifically the endoscopic probe which is the most expensive part and therefore at least some parts of the assembly are advantageously reusable.

According to a second aspect of the present disclosure, which may be provided in addition to the first aspect or in alternative thereto, assemblies according to the present disclosure advantageously further comprise an attachment module. The attachment module comprises a connector system configured to secure the attachment module to a support, such as the handle of an endoscope and advantageously to provide access to the instrument channel of the endoscope. The attachment module can further comprise a telescopic tubular body extending from a first end to a second end opposite the first end, and defining an internal channel of the attachment module, which is sized to slidingly receive the catheter body. The connector system is advantageously provided at the second end.

Advantageously, the attachment module further comprises a handle member and a first locking system attached to the telescopic tubular body at the first end and configured to lock a motion of the guiding catheter relative to the handle member in respect of at least one of: a translation along a longitudinal axis of the telescopic tubular body and a rotation about the longitudinal axis. Advantageously, the guiding catheter comprises a first connector member, which can be arranged at the proximal end of the guiding catheter, and configured to advantageously releasably interlock with a corresponding connector member of the first locking system so as to enable an integral motion of the guiding catheter and the handle member. The attachment module can further comprise a second locking system configured to fixedly secure the guiding catheter axially relative to the connector system and/or the support, which second locking system can be arranged at the second end.

One advantage of assemblies according to the second aspect is that they facilitate extended navigation beyond the reach of conventional endoscopes. Specifically, the attachment module provides an easier yet more precise manipulation of the guiding catheter which facilitates distal navigation of the guiding catheter and the endoscopic probe and thereby improved visualisation of the anatomical structures, enabling improved diagnostics.

Advantageously, the assembly further comprises an endoscope, including an endoscope handle connected to an endoscope body. The endoscope body comprises an instrument channel (commonly referred to as working channel), wherein the endoscope handle comprises an entry port communicating with the instrument channel, wherein the guiding catheter is configured to be slidingly received in the instrument channel and through the entry port. The endoscope can further comprise a second imaging device arranged at a distal end of the endoscope body. The endoscope handle can form the support and can comprise a connector part configured to co-operate with the connector system to attach the attachment module to the endoscope handle.

In another aspect, methods of performing endoscopy are disclosed herein, particularly for performing endoscopy in the pulmonary tract. These methods utilize assemblies according to the present disclosure. In a first operation, the guiding catheter is inserted in an anatomical cavity or lumen. The guiding catheter can be inserted directly or via the instrument channel of an endoscope, to which the attachment module of assemblies can be mounted. In a second, optional operation, the guiding catheter is deployed distally beyond a distal tip of the endoscope, possibly by manipulating handle member of the attachment module and to which guiding catheter is attached. In a third operation, guiding catheter is navigated through the anatomical cavity or lumen. The guiding catheter can e.g. be navigated beyond the distal tip of the endoscope. In a fourth operation, images are taken with the imaging device at the distal tip of the endoscopic probe. These images can be displayed on a visual display at the proximal side. In a fifth, optional operation, the guiding catheter can be fixedly secured in position relative to the endoscope by operating the second locking system of the attachment module, e.g. when the guiding catheter and/or endoscopic probe reaches the anatomical target location. In a sixth operation, an external part, such as a lens or screen, of the imaging device of endoscopic probe may get soiled or smudged by body fluids while navigating, impairing proper visualization. Fluid is supplied via the second inlet port of the fluid delivery module (e.g. by operating the flow control valve) to the internal lumen which flushes the distal tip of the endoscopic probe and cleans it. To this end, the endoscopic probe can be retracted until the imaging device (distal portion) is completely received inside the internal lumen of the guiding catheter, is flush with the distal tip of the guiding catheter, or is at least partially extending from the distal tip. The imaging device is now ready to be deployed again at or even beyond the outlet aperture of the guiding catheter. In a seventh operation, which can be performed in addition or alternatively to the sixth operation, the endoscopic probe is retracted inside the internal lumen, advantageously such that the imaging device is completely within the internal lumen of the guiding catheter, i.e., the imaging device is at a proximal side of the outlet aperture of the internal lumen. As a result, a porthole effect is created allowing an anatomical structure to be suitably imaged by the imaging device while being in the internal lumen. Optionally, the distal tip or outlet aperture of the catheter body is held against the anatomical target structure. By so doing, the distal tip of the catheter body advantageously maintains the anatomical structure in a desired position while being imaged by the imaging device. In an eighth, optional operation, a diagnostic or surgical procedure is executed, which may include taking a measurement of an anatomical structure. In addition or alternatively, the endoscopic probe is removed from the guiding catheter and a surgical (e.g., biopsy) instrument deployed through the guiding catheter. It will be appreciated that the order of the operations described above is illustrative and can be changed, or some operations executed simultaneously.

Another method of performing endoscopy, particularly endoscopy in the pulmonary tract or bronchoscopy, according to the present disclosure, comprises navigating an endoscope device through an anatomical cavity or lumen until arriving at an anatomical park position and deploying the guiding catheter according to aspects of the present disclosure through an instrument channel of the endoscope. The guiding catheter can be deployed beyond the distal tip of the endoscope, e.g. to arrive at an anatomical target location, and its position is secured or fixed with respect to a reference, which can be the endoscope or a fixed reference attached to or external to the patient. The endoscopic probe is deployed through the guiding catheter to image the anatomical target location. To do so, the endoscopic probe can be deployed beyond the outlet aperture of the guiding catheter, or alternatively retracted from the outlet aperture to create a visual porthole effect for imaging the anatomical target location.

Advantageously, a navigation route along the anatomical cavity or lumen is determined, e.g. based on medical imaging technologies, and the endoscope, the guiding catheter, and/or the endoscopic probe are navigated along the navigation route. Advantageously, a trackable marker, particularly an electromagnetic sensitive device, is tracked along the navigation route. The trackable marker can be integrated in the endoscope, the guiding catheter, and/or the endoscopic probe.

Advantageously, a virtual model of the anatomical cavity or lumen is determined. The virtual model is mapped to images captured with the endoscope device and/or the endoscopic probe based on a position determined through the trackable marker.

A tracking probe comprising the trackable marker is deployed through the endoscope and/or the guiding catheter and the endoscope and/or guiding catheter including the tracking probe are navigated along the navigation route. The tracking probe can be retracted from the guiding catheter, specifically when the guiding catheter is at the anatomical target location, as may be determined by tracking the trackable marker, and advantageously a (distal) position of the guiding catheter is fixed. With the tracking probe removed, the endoscopic probe according to aspects of the present disclosure can be deployed through the (internal lumen of the) guiding catheter and the anatomical target location is imaged with the imaging device of the endoscopic probe. A visual inspection of the anatomical target location can be performed based on images captured by the imaging device. In addition, or alternatively, a surgical instrument is deployed through the guiding catheter and a surgical intervention, such as a biopsy, is performed at the anatomical target location.

Alternatively, the endoscopic probe according to aspects of the present disclosure can comprise a trackable marker and can act as the tracking probe in the above methods.

In the methods according to the present disclosure the endoscopic probe hence advantageously allows to perform additional visual inspection of an anatomical target location to validate, by close visual inspection, a condition and/or a position of the anatomical target location as determined by a medical imaging technique. This can be beneficial so as to properly assess the condition and/or position prior to performing a surgical intervention, such as a biopsy.

### Brief description of the figures

Aspects of the present disclosure will now be described in more detail with reference to the appended drawings, wherein same reference numerals illustrate same features and wherein:
Figure 1 represents a plan view of an embodiment of an assembly according to the present disclosure;
Figure 2 represents a top view of the assembly of Fig. 1;
Figure 3 represents a longitudinal cross section view along section line A-A of the assembly of Figs. 1 and 2;
Figure 4 represents a perspective view of the distal end of the assembly of Fig. 1;
Figure 5A represents a longitudinal cross section view of the distal end of the assembly of Fig. 1 in which the endoscopic probe is deployed beyond the distal end of the catheter body; Figure 5B represents a longitudinal cross section view of the distal end of the assembly of Fig. 1 in which the endoscopic probe is completely received in the internal lumen of the catheter body;
Figure 6 represents a perspective view of another embodiment of an assembly according to the present disclosure, which incorporates the assembly of Fig. 1;
Figure 7 represents a longitudinal cross section view of the assembly of Fig. 6;
Figure 8A represents a perspective view of the attachment module comprised in the assembly of Fig. 6; Figure 8B represents another perspective view of the attachment module of Fig. 8A;
Figure 9 represents a perspective view of the assembly of Fig. 6 attached to the proximal handle of an endoscope;
Figure 10 represents a perspective view of the distal end of an endoscope in which the distal end of the catheter of the assembly of Fig. 6 is deployed beyond the distal end face of the endoscope;
Figure 11 represents a flow diagram of operations of a method of performing endoscopy according to the present disclosure;
Figure 12 represents a computer tomography image of a human lung in a transverse plane;
Figure 13 represents an image of a virtual reconstruction of a bronchus;
Figure 14 represents an image of a virtual reconstruction of an internal part of the bronchus of Fig. 13;
Figure 15 represents an endoscope image of the internal part of the bronchus corresponding to Fig. 14;
Figure 16 represents an image taken by the endoscopic probe according to aspects of the present disclosure.

### Detailed Description

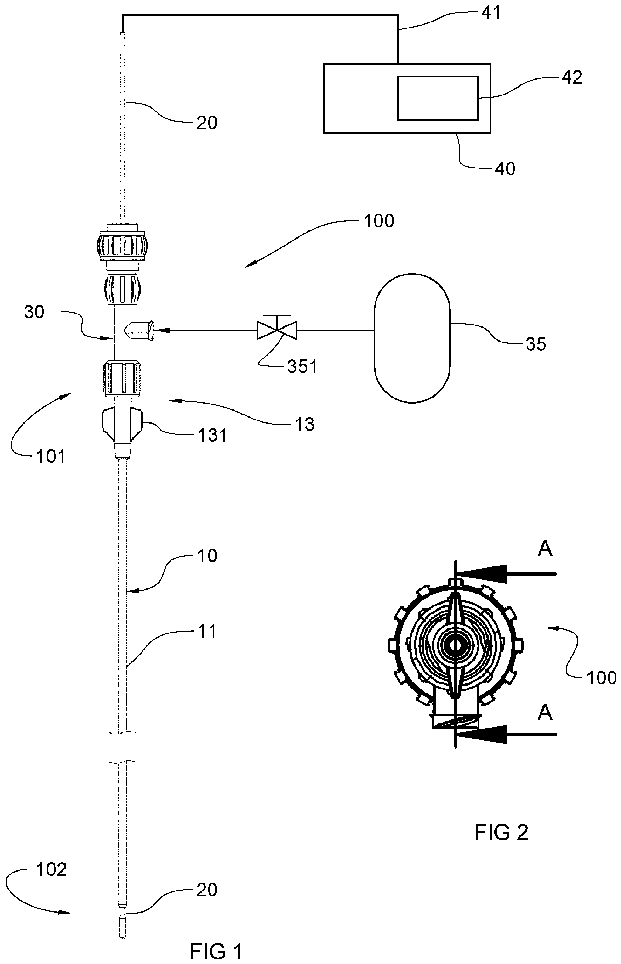

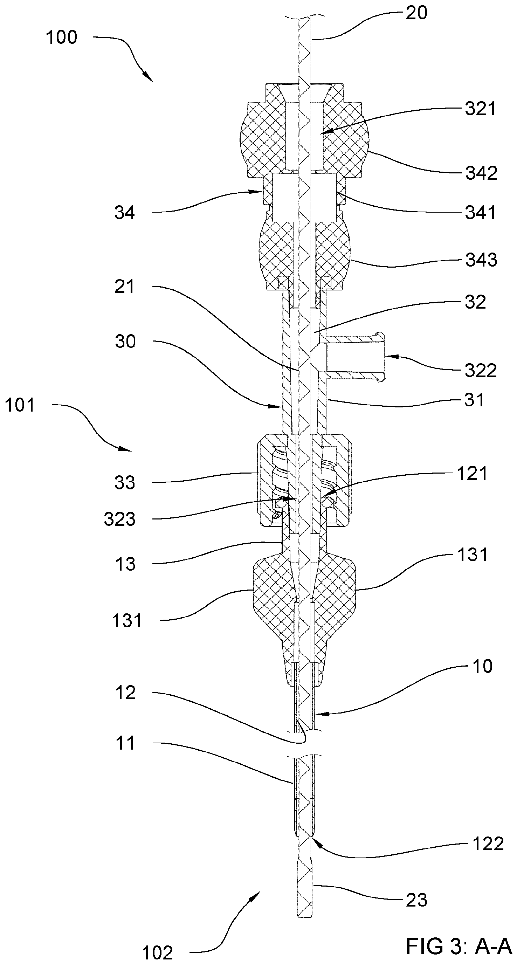

Referring to Figs. 1-3, an exemplary assembly 100 comprises a catheter 10, a probe 20 and a fluid delivery module 30. Catheter 10 extends from a proximal end 101, e.g. corresponding with a side proximal to the operator, to a distal end 102, e.g. that is configured to reach an anatomical target location that is to be visualized. It will be appreciated that the terms "proximal" and "distal" are utilized herein with reference to the operator that manipulates the assembly.

Catheter 10 comprises an elongate flexible catheter body 11 which may extend from the proximal end 101 to the distal end 102. Catheter body 11 is tubular and comprises an internal lumen 12 reaching from an inlet aperture 121 at the proximal end 101 to an outlet aperture 122 at the distal end 102.

The catheter body 11 can be configured to be inserted in a natural or surgically created anatomical orifice. Advantageously, as will be described in further detail below, catheter body 11 is configured to be inserted in an instrument channel of a conventional endoscope. The outer diameter of catheter body 11 is advantageously 3.0 mm or smaller, advantageously between 1.2 mm and 2.8 mm, and advantageously between 1.5 mm and 2.8 mm.

The catheter body 11 is advantageously made of, or comprises, a radio-opaque material to facilitate its localization, e.g. through external imaging techniques such as computer tomography or fluoroscopy. Advantageously, the catheter body 11 is configured to transmit torque between the proximal end 101 and the distal end 102. An efficient torque transmission can be achieved by providing a suitable reinforcement braid in the wall of the catheter body 11. By so doing, a rotation about the longitudinal axis of catheter body 11 effected at the proximal end 101 can be transmitted to the distal end 102 to enable navigation within an anatomical cavity. Alternatively, or in addition, the catheter can comprise an actuation system, e.g. comprising cables or the like, to make catheter body steerable. It is additionally or alternatively possible to make the catheter body with a pre-formed shape, such as a bent, waved or other appropriate shape to ease navigation.

The catheter body 11 is advantageously straight at the distal end 102, although slightly bent shapes can be contemplated. Outlet aperture 122 of the internal lumen 12 is advantageously arranged at the distal tip of catheter body 11. The distal tip is advantageously atraumatic to prevent damaging tissue while navigating through an anatomical cavity. By way of example, an outer circumferential edge of the distal tip of catheter body 11 can be rounded and/or made of a softer, e.g. resilient, material. In some examples, the reinforcement braid in the wall of the catheter body extends to somewhat before the distal tip, and the distal tip is advantageously free from reinforcement.

At the proximal end 101, catheter 10 can comprise a connector module 13, e.g. comprising a screw connector or Luer-lock fitting, to fluid tightly connect catheter 10 to fluid delivery module 30 in an advantageously releasable manner. Connector module 13 can comprise the inlet aperture 121. A proximal portion of the internal lumen 12 can extend through connector module 13 until continuing through the catheter body 11. Connector module 13 can further comprise one or more locking members 131, the operation of which will be described further below.

Fluid delivery module 30 comprises a tubular body 31 with internal channel 32. It comprises a first inlet port 321, a second inlet port 322 and an outlet port 323, all communicating with channel 32. A connector 33 co-operates with connector module 13 of catheter 10 to secure the catheter 10 and the fluid delivery module to one another in a fluid tight manner. As a result, channel 32 of fluid delivery module 30 communicates with internal lumen 12 of catheter 10 through outlet port 323 and inlet aperture 121. It is alternatively possible to manufacture catheter 10 and fluid delivery module 30 as a single integral structure, eliminating connectors 13 and 33.

First inlet port 321, which may be coaxially aligned with outlet port 323, is configured to accept insertion of the endoscopic probe 20. Probe 20 is inserted through channel 32 via the first inlet port 321, and further deployed through internal lumen 12 via outlet port 323 and inlet aperture 121. A seal system 34 is advantageously provided at, or in connection with, the first inlet port 321. Seal system 34 comprises a sealing member 341 configured to fluid tightly seal the channel 32 and/or the first inlet port 321 when the endoscopic probe 20 is inserted therethrough. Sealing member 341 can be formed of a soft or resilient, e.g. elastomeric, material. Sealing member 341 can comprise a through-bore configured to slidingly receive the probe body 21. Optionally, sealing member 341 can be accommodated in one or more co-operating, e.g. threaded, connector members 342, 343 which are configured to tighten and release the sealing member 341 as known in the art.

The second inlet port 322 communicates with channel 32 at a position advantageously downstream of the first inlet port 321. The second inlet port 322 is configured as a fluid delivery port and/or a fluid suction port. In use, the second inlet port 322 can be coupled to an external fluid source 35. Advantageously, a valve 351 (Fig. 1), such as a flow control valve is provided in fluid communication with the second inlet port 322. Valve 351 can be connected to fluid source 35 and can be operated to deliver fluid to the internal lumen 12 when needed. By way of example, valve 351 is a normal-closed valve and comprises an interface, such as a control knob or press button as known in the art, which when operated is configured to open a flow passage of valve 351 to supply fluid from the fluid source 35 to the second inlet port 322 and further to internal lumen 12. Fluid source 35 can comprise a gas, such as CO₂ or air, or a liquid, e.g. a saline solution, a physiological solution, or a contrast agent, the latter being particularly suitable for application in the biliary duct. The fluid source 35 can be a syringe. In that case, valve 351 can be omitted.

With endoscopic probe 20 inserted and sealing member 341 tightened against the probe 20, a fluid can be delivered to internal lumen 12 through the second inlet port 322. This allows to supply fluid to the internal lumen 12 while preventing fluid to egress from the first inlet port 321.

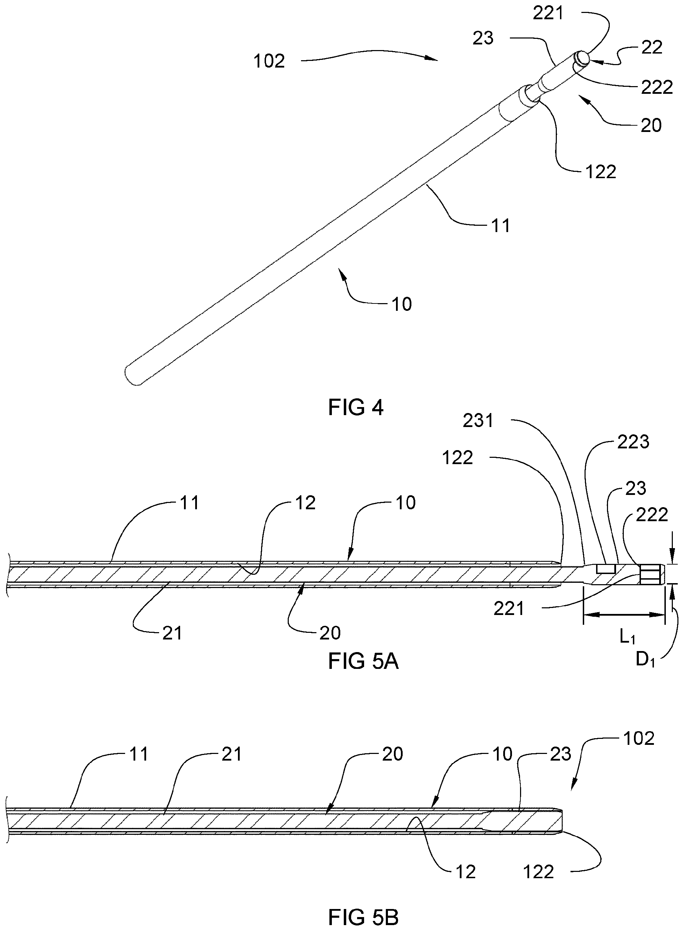

Referring to Figs. 4 and 5A-5B, the endoscopic probe 20 comprises an elongate and flexible probe body 21. Probe body 21 has a length advantageously larger than a length of the catheter body 11, allowing full insertion through the internal lumen 12 of the catheter 10, from the proximal end 101 to the distal end 102, and possibly deployment of the endoscopic probe 20 beyond the distal tip (outlet aperture 122) of catheter 10.

An imaging device 22, advantageously a video imaging device, is provided at the distal end of the endoscopic probe 20. The imaging device 22 can comprise a camera 221, advantageously provided at the distal tip face of the endoscopic probe 20 and advantageously configured to capture visual images. Camera 221 can be a CMOS (Complementary metal-oxide-semiconductor) image sensor, such as an Omnivision^{™} image sensor, a fiber optic imaging system, or any other suitable lightbased imaging system, e.g. capable of capturing visible light and/or infrared light. Camera 221 advantageously comprises an image sensor having a size of 1 mm by 1 mm or less. Camera 221 can further comprise one or more lenses, e.g. that form the distal tip face of endoscopic probe 20. The imaging device can further comprise a light source 222, such as provided by an optical fiber or a light emitting diode (LED). By way of example, light source 222 can be arranged coaxially with an optical axis of the camera 221. Light source can be arranged circumferential to the camera 221.

Referring back to Fig. 1, the imaging device 22 is connected by suitable wiring 41, e.g. optical fiber and/or electrically conducting wire or cable, to an external control unit 40. Control unit 40 can comprise a visual display 42 to display images taken by the imaging device 22.

Referring again to Fig. 4 and Figs. 5A-5B, the imaging device 22 can be housed in a thickened distal portion 23 of the endoscopic probe 20. Distal portion 23 is attached to the probe body 21 at the distal end of the probe body 21. The distal portion 23 can have substantially cylindrical or spherical shape, or any other suitable shape, e.g. with rotational symmetry about the longitudinal axis.

The internal lumen 12 advantageously has a diameter between 0.9 mm and 2.6 mm, advantageously between 1.2 mm and 2.4 mm, such as 2.1 mm. The distal portion 23 advantageously has a cross sectional size, e.g. a diameter, larger than a diameter of the probe body 21. An external diameter D₁ of the distal portion 23 is advantageously between 0.8 mm and 2.4 mm, advantageously between 1.0 mm and 2.0 mm. While the diameter D₁ is smaller than the diameter of the internal lumen 12 of catheter 10 to allow deployment of the distal portion 23 throughout the internal lumen 12 (Fig. 5B), it will be appreciated that the probe body 21 can have a considerably smaller diameter, e.g. between 0.5 mm and 1.8 mm, advantageously between 0.6 mm and 1.5 mm. A ratio of diameter of probe body 21 to the diameter of internal lumen 12 is advantageously between 0.15 and 0.9, advantageously between 0.3 and 0.75. A ratio of diameter D₁ of the distal portion 23 to the diameter of internal lumen 12 is advantageously between 0.3 and 0.95, advantageously between 0.5 and 0.9. The distal portion 23 can alternatively have a same diameter as the probe body 21. An axial length L₁ of distal portion 23 is advantageously as small as possible while allowing to accommodate parts of the imaging device 22. Suitable axial lengths L₁ of the distal portion 23 are between 3 mm and 10 mm.

By reducing the diameter of the probe body 21 compared to the diameter of the distal portion 23, e.g. utilizing the ratios indicated above a larger cross section of the internal lumen is made available for fluid flow. Hence, with the endoscopic probe 20 inserted in catheter 10, fluid can be delivered through the internal lumen from the second inlet port 322 of the fluid delivery module 30 with greater ease, obtaining a smaller fluid pressure drop for a given flow rate, or enabling a larger flow rate for a given pressure drop along the internal lumen 12.

Advantageously, a ratio of diameter D₁ of the distal portion 23 to axial length L₁ of the distal portion is advantageously between 0.1 and 0.4, advantageously between 0.2 and 0.3, particularly between 0.23 and 0.27. This ratio ensures optimal navigation of the endoscopic probe 20 through the internal lumen 12, particularly when the catheter body has curved or bent portions, e.g. when inserted in an anatomical cavity. For larger values of L₁, the diameter D₁ should be selected smaller to enable proper navigation through the possible bends of internal lumen of the catheter body. When on the other hand L₁ is smaller, the diameter D1 can be selected larger.

Probe body 21 advantageously accommodates only the wiring 41 and a suitable sheath with or without a reinforcement. Advantageously, probe body 21 is free from internal channels with distal outlet apertures, such as fluid supply and/or suction channels, or instrument channels, making it of compact dimensions and simple structure, which furthermore is easily cleanable and sterilizable for use on multiple patients. This allows to decrease the cost of the endoscopic probe per intervention. Alternatively, the probe body 21 can comprise one or more of such internal channels if size limitations allow. The probe body 21 can comprise a reinforcement, possibly embedded in the sheath, providing suitable axial stiffness for deploying the endoscopic probe through the catheter 10 (internal lumen 12). The reinforcement can comprise or consist of a braid, which can additionally be configured to transmit torque and/or torsion or rotation for continued navigation beyond the outlet aperture 122 of the catheter 10. The distal tip of the distal portion 23 can be made atraumatic, e.g. by proper filleting or rounded shape of the distal tip.

Additionally, a thickened distal portion 23 compared to the diameter of the probe body 21 creates a shoulder or flange 231, advantageously facilitating advancement of the endoscopic probe 20 through the internal lumen 12 while flushing the internal lumen with a fluid from the second inlet port 322.

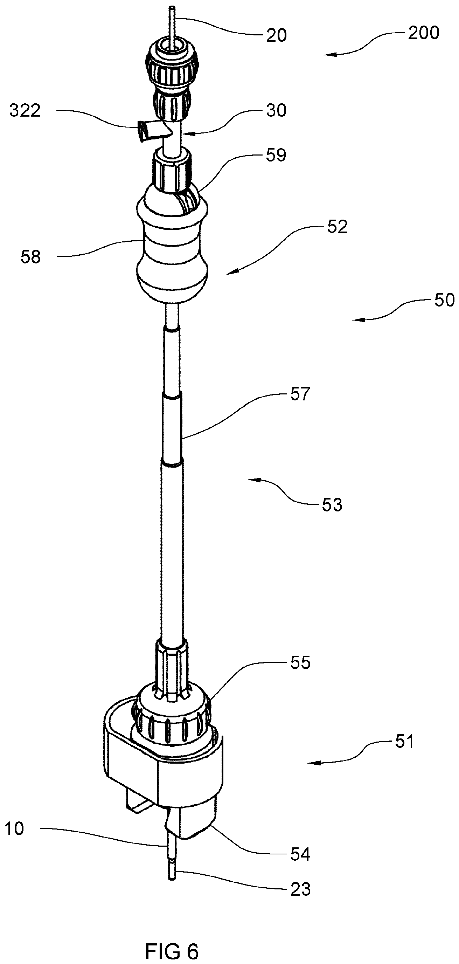

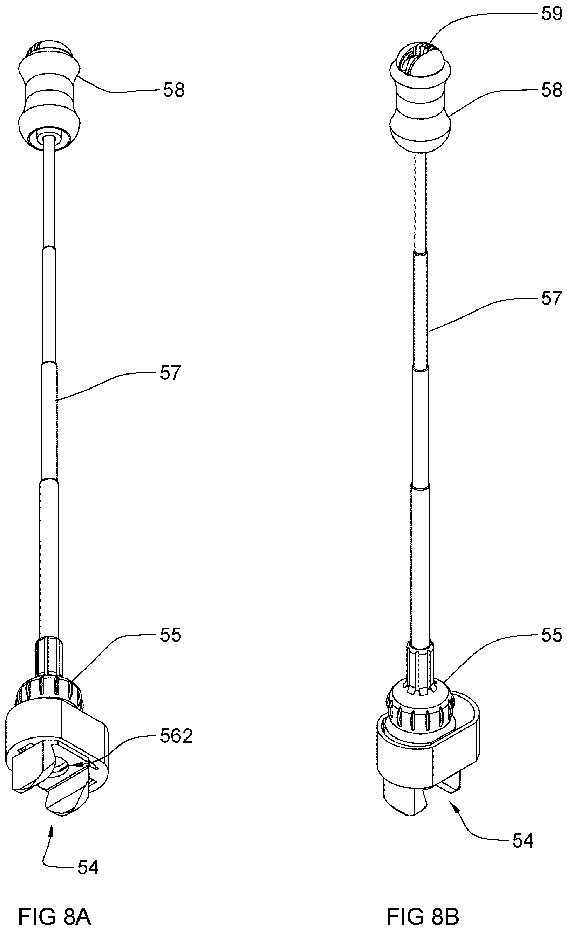

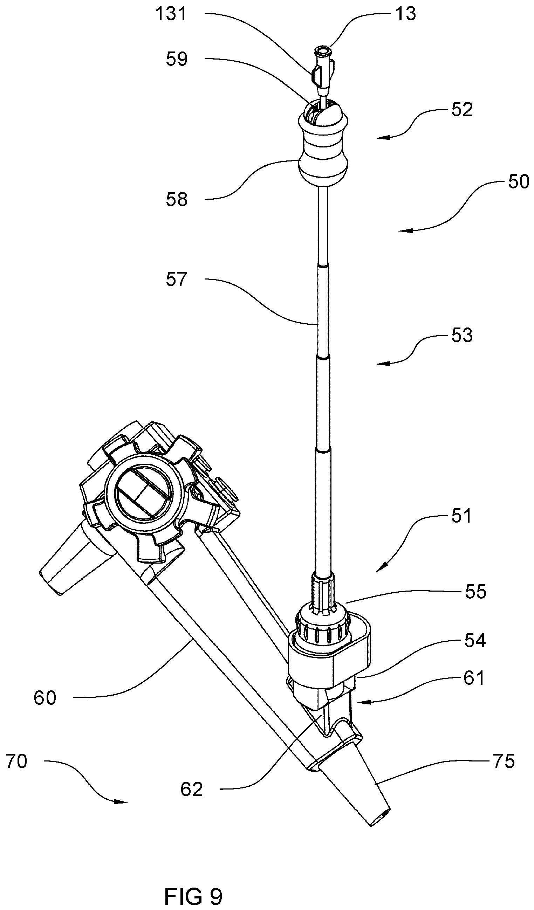

Referring to Figs. 6-9, another exemplary assembly 200 comprises assembly 100 and further comprises an attachment module 50 configured to attach the assembly 100 to the handle 60 of an endoscope 70. Attachment module 50 comprises a distal part 51, a proximal part 52, and a connecting part 53, connecting the distal part 51 to the proximal part 52.

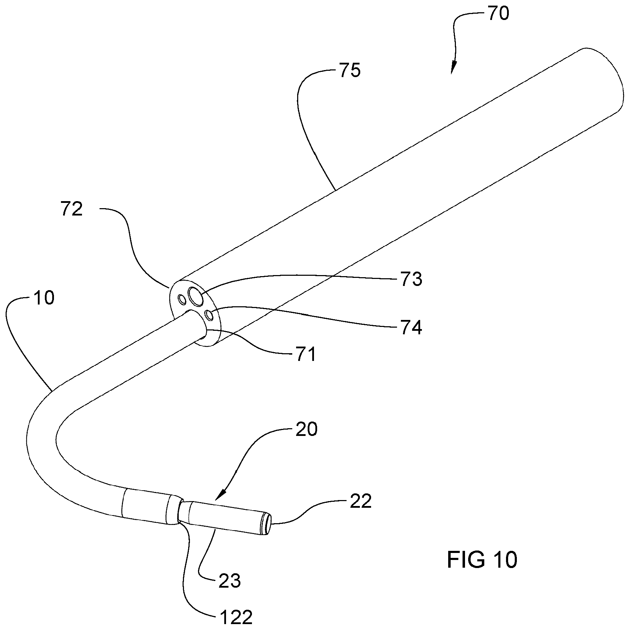

The distal part 51 of attachment module 50 comprises a connector system 54 configured to secure or attach the (distal part 51 of) attachment module 50 to the handle 60. Endoscope handle 60 is connected to an elongate body 75 of endoscope 70 and provides access to an instrument channel 71 of the endoscope (Fig. 10). The connector system 54 mates or locks with a co-operating connector part 62 provided on handle 60, adjacent, e.g. circumferential, to an entry port 61 of the handle 60 that communicates with the instrument channel 71. Connector system 54 is configured to secure the (distal part 51 of) attachment module 50 relative to the entry port 61 of handle 60 at least in respect of an axial (translational) motion degree of freedom, e.g. through a snap-fit connector. The connector system 54 can additionally be configured to secure the (distal part 51 of) attachment module 50 rotationally, e.g. in one or more rotational degrees of freedom with respect to the handle 60.

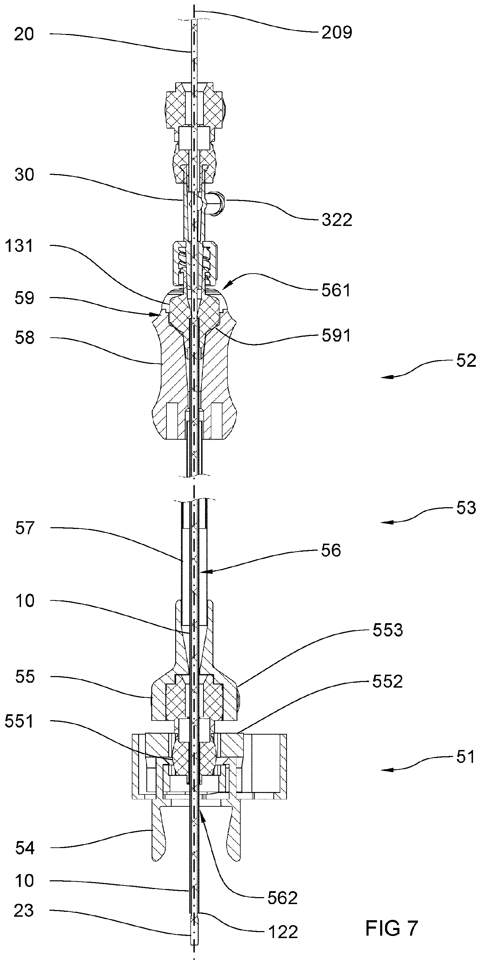

Attachment module 50 comprises an internal channel 56 extending from the proximal part 52, through the connecting part 53, to the distal part 51. An entry port 561 (Fig. 7) at the proximal part 52 provides access to the internal channel 56. An exit port 562 at the distal part 51 provides an outlet of the internal channel 56 and access from the internal channel 56 to the instrument channel 71 of endoscope 70. Exit port 562 can be coaxially aligned with connector system 54. Internal channel 56 has a diameter sized to allow catheter 10 passing through. Catheter 10 is inserted with the distal end 102 through inlet port 561 and advanced until it reaches beyond exit port 562, where catheter 10 enters the instrument channel 71 of endoscope 70. With reference to Fig. 10, catheter 10 is further advanced through instrument channel 71 until it reaches beyond the distal tip 72 of endoscope 70. The endoscope 70 can comprise a camera 73 or other imaging sensor configured to capture visual images, arranged at the distal tip 72. One or more light sources 74 can be provided at the distal tip as well.

Referring again to Figs. 6-9, a handle member 58 is provided at the proximal part 52 of attachment module 50. Handle member 58 is fixedly attached to the proximal end of a telescopic tubular body 57, the length of which is extendable from the distal part 51 in proximal direction. Telescopic tubular body 57 advantageously forms the connecting part 53, connecting handle member 58 to the distal part 51 (exit port 562) and defining the internal channel 56.

Handle member 58 can be manipulated by an operator as will be described below. A locking connector 59 can be provided at the proximal part 52, advantageously circumferential to entry port 561, and advantageously integrated in handle member 58. Locking connector 59 co-operates with one or more locking members 131 of connector module 13 of catheter 10 to lock a motion of the catheter 10 with respect to the handle member 58 in respect of one or more degrees of freedom. Particularly, the locking members 131, advantageously shaped as wing shaped tabs, are configured to snugly engage in corresponding slots 591 of locking connector 59 to lock rotation of the catheter 10 about longitudinal axis 209 with respect to the handle member 58 and/or to lock translation of the catheter 10 along longitudinal axis 209 with respect to handle member 58. Locking connector 59 advantageously provides a releasable connection of locking members 131, e.g. a snap-fit connector.

When the locking members 131 of catheter 10 are locked into locking member 59, an operator can easily apply a rotation and/or axial motion to the catheter 10 by manipulating handle member 58. By way of example, pushing handle member 58 towards the distal side will cause the members of telescopic tubular body 57 to collapse into one another, and consequently causes the catheter 10 to advance further distally inside the instrument channel 71 of endoscope 70. In addition, or alternatively, handle member 58 can be rotated about longitudinal axis 209 with respect to endoscope handle 60 (and hence with respect to the connector system 54 / distal part 51), which will cause a rotation or torque to be imparted to the catheter 10 at the proximal side, which can be transmitted through the structure of the catheter body 11 towards the distal end 102. The attachment module 50 therefore can facilitate manipulation and navigation of the assembly, particularly beyond the distal tip of the endoscope 70.

Attachment module 50 can further comprise a locking system 55, configured to secure or lock motion of the guiding catheter 10 relative to the distal part 51, particularly relative to connector system 54 and/or instrument channel 71 in respect of one or more degrees of freedom. In one example, locking system 51 is configured to lock or clamp a translation degree of freedom of the guiding catheter 10 along the longitudinal axis 209 relative to the distal part 51 of attachment module 50 and/or instrument channel 71. The rotational degree of freedom of guiding catheter 10 about the longitudinal axis 209 relative to the distal part 51 / instrument channel 71 can be left free or can be locked in addition by locking system 55.

In a specific example, locking system 55 can comprise a sleeve 551 formed of a soft or resilient, e.g. elastomeric, material and having a through bore configured to receive guiding catheter 10 through it. The sleeve 551 is accommodated in an enclosure 552 which e.g. can have a conical recess accepting sleeve 551. A member 553 is rotatable relative to enclosure 552 thereby applying a force on sleeve 551 that acts to reduce the diameter of the through bore of sleeve 551, e.g. by deforming sleeve 551. Member 553 can be configured to rotate about longitudinal axis 209, although other configurations and rotation axes are possible as known in the art. Locking system 55 is advantageously releasable.

Locking system 55 is convenient when the guiding catheter 10 has reached the final or target location. Locking system 55 can then be operated to fixedly secure the (axial) position of the guiding catheter 10 relative to the instrument channel 71 and the distal tip of endoscope 70. This avoids that any inadvertent manipulation of the handle member 58 unwishfully moves the catheter 10 away from its desired position.

The guiding catheter 10, the fluid delivery module 30 and the attachment module 50 can be formed of one or a combination of polymeric and/or synthetic materials, such as medical grade silicone materials, and medical grade thermoplastic materials, such as polypropylene or polycarbonate. At least some portions of the guiding catheter can be disposable. The endoscopic probe 20 is advantageously formed of materials that can be cleaned and/or sterilized between uses on different patients. At least some parts of the endoscopic probe 20 can be reusable for uses on different patients.

At least some portions of the catheter body 11 and/or the endoscopic probe 20 can be formed or comprise a radio-opaque material to ease its localization. Referring to Fig. 5A, the catheter body 11 and/or the endoscopic probe 20 can comprise one or more trackable markers 223 that allow (initial) tracking via electromagnetic navigation, possibly in combination with fluoroscopic imaging as known in the art. The trackable marker is advantageously an electromagnetically sensitive device that is arranged in or on any one of the catheter body 11, the probe body 21 and the distal portion 23 of the endoscopic probe. Electromagnetic navigation can be utilized in the present disclosure in combination with visual guidance by the imaging device 22 to improve positioning accuracy and orientation within the body.

The probe body 21 advantageously has suitable axial stiffness or rigidity to prevent buckling while the endoscopic probe is advanced or deployed though the internal lumen 12 of the guiding catheter 10. In some examples, the probe body 21 can comprise a central wire made of a nickel titanium alloy (nitinol), or other suitable metal alloy and a sheath. The central wire advantageously forms a core of the probe body and is arranged inside the sheath. Such a central wire can impart appropriate stiffness and buckling resistance to the probe body. Electrical and optical wires and cables can be arranged inside the sheath as well. The central wire and the sheath can extend substantially along the entire length of the probe body, such as from the proximal end to the distal end thereof.

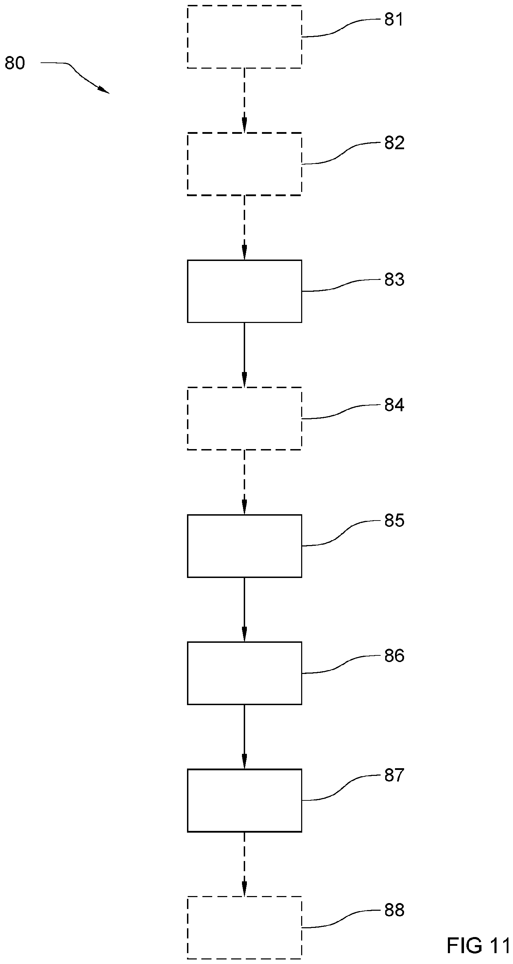

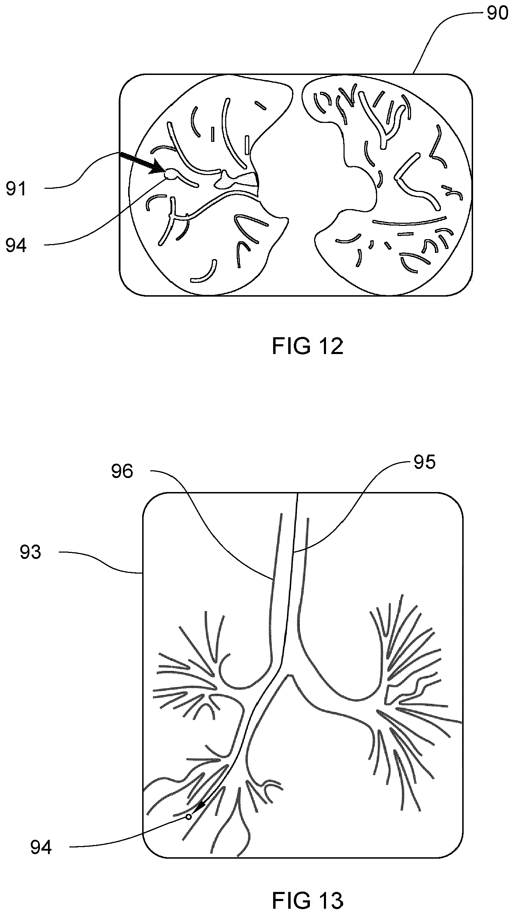

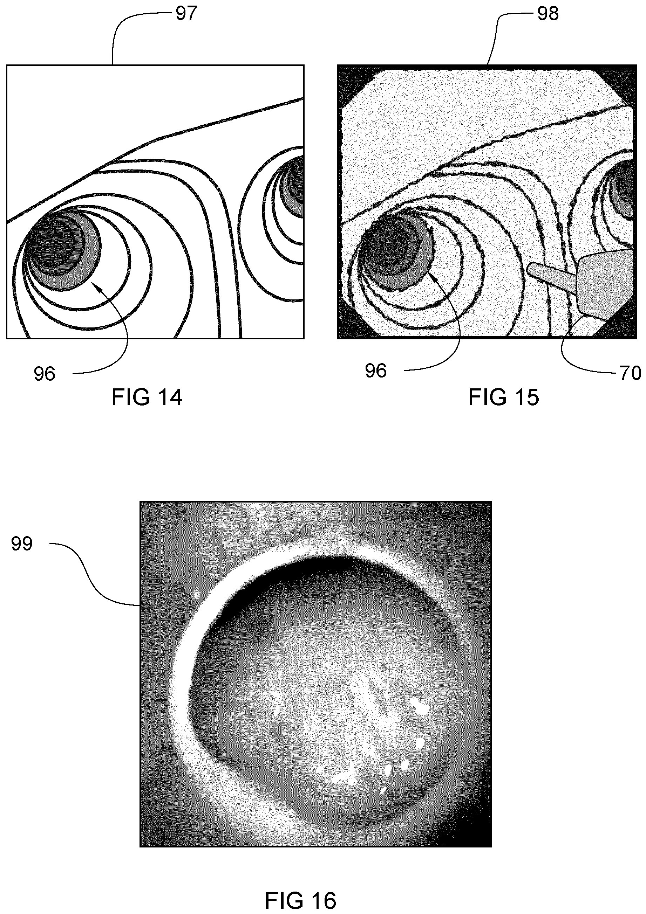

Referring to Figs. 11-16, a method 80 of performing endoscopy utilizing assemblies as disclosed herein can start with an operation 81 in which a lesion, such as a pulmonary nodule (Fig. 12, arrow 91) is uncovered in a patient. The lesion can be uncovered by a medical imaging examination, such as by Computer Tomography (CT) or fluoroscopic imaging of the anatomical cavity or lumen of interest, such as a CT image 90 of the lung. A medical practitioner may wish to further examine the lesion and/or perform a biopsy to investigate its malignity. This requires an endoscopic intervention. In operation 82, an endoscopic navigation is planned. Operation 82 can start with generating a virtual model 93 of the anatomical cavity or lumen 96, e.g. of the bronchi, which can be obtained through the medical imaging examination performed in operation 81. A navigation route 95, e.g. from the trachea through the bronchi, to arrive at the lesion 94 defined from operation 81 can be planned based on the virtual model 93.

At operation 83, an endoscope 70, such as a bronchoscope, is utilized to perform navigation through the anatomical cavity or lumen 96 along the planned route 95.The endoscope 70 can be inserted in the anatomical cavity or lumen 96, via an anatomical or surgically created artificial orifice. The endoscope 70 is guided through the anatomical cavity or lumen 96 via endoscopic vision provided by camera 73 and light source 74 of endoscope 70.

At operation 84, the virtual model 93 of the anatomical cavity or lumen 96 can be mapped to the real anatomical cavity or lumen 96 by mapping the virtual images 97 to the endoscope images 98 of the real anatomical cavity or lumen. To this end, a trackable marker can be utilized for tracking the position of the endoscope as it is advanced along navigation route 95. The trackable marker, such as an electromagnetic marker, can be provided on the endoscope, or on a probe or surgical instrument that is inserted through the endoscope. The probe can be inserted in a guiding catheter, such as guiding catheter 10, and the guiding catheter inserted in the instrument channel 71 of the endoscope 70. The position of the trackable marker can be tracked by non-contact measurement with respect to a fixed reference according to known methods. From the tracked position of the endoscope, the position of images captured by the endoscope can be determined, allowing to map the virtual image 97 to the endoscope image 98.

At operation 85, the distal tip 72 of the endoscope has arrived at an anatomical park position, where it cannot reach beyond, e.g. because the anatomical structure is too small, too rigid, and/or too complex for passing through. If not done yet, the guiding catheter 10 is inserted in the instrument channel 71 of the endoscope 70. The guiding catheter 10 can be further deployed (distally) beyond the distal tip of the endoscope, e.g. as shown in Fig. 10, and advanced along navigation route 95. Advancement of the guiding catheter 10 can be tracked by a trackable marker provided on the guiding catheter or a tracking probe inserted in the guiding catheter. The tracking probe can be endoscopic probe 20 according to the present disclosure or a different probe.

Optionally, the attachment module 50 of assembly 200 is mounted on the handle 60 of endoscope 70 and the guiding catheter 10 is inserted through the entry port 561 and via internal channel 56 to an entry port of the handle 60 and further deployed through the instrument channel 71 of the endoscope 70 until the distal end 102 of guiding catheter 10 reaches the distal tip 72 of endoscope 70. Locking members 131 of connector module 13 can be brought in locking engagement with the locking member 59. The members of telescopic tubular body 57 can be completely extended in this position.

To navigate the guiding catheter, the handle member 58 can be manipulated, while members 59 and 131 are in locking engagement, to move it in distal direction (towards distal part 51 and handle 60). By so doing, the members of telescopic tubular body 57 get collapsed, causing advancement of the guiding catheter 10 through the instrument channel 71 and further distally beyond the distal tip 72. Optionally, handle member 58 can be rotated about longitudinal axis 209 and the rotation transmitted to the guiding catheter 10 through members 59 and 131. This may impart a rotation of the distal end 102 of the guiding catheter 10 / catheter body 11, which may facilitate navigation.

At operation 86, the guiding catheter 10 arrives in proximity of the location of lesion 94, as may be determined through tracking as described above. Advantageously, the guiding catheter 10 is fixed in position with respect to the endoscope and/or with respect to the fixed (patient) reference. The guiding catheter 10 can be fixedly secured in position relative to the endoscope 70 by operating locking system 55. This allows handle member 58 to be released and possibly locking members 131 released from connector member 59. If a tracking probe other than endoscopic probe 20 is utilized for navigation, the tracking probe is removed from the guiding catheter and the endoscopic probe 20 is inserted instead.

The endoscopic probe 20, e.g. if it comprises a trackable marker, can alternatively be pre-inserted in the internal lumen 12 of the catheter 10 while the catheter body 11 is deployed through the instrument channel 71 of the endoscope. Alternatively, the endoscopic probe 20 can be inserted successively. While endoscopic probe 20 is deployed through the internal lumen 12 of guiding catheter 10, pressurized fluid, which can be a gas such as CO₂ or a liquid such as a saline solution, can be supplied to the internal lumen at a suitable pressure, particularly via the second inlet port 322 of fluid delivery module 30. Supplying some fluid while deploying the endoscopic probe 20 can facilitate advancement of the endoscopic probe 20 through the internal lumen 12, particularly when the endoscopic probe 20 is provided with a thickened distal portion 23.

At operation 87, the guiding catheter 10 and/or endoscopic probe 20 is at the anatomical target location, i.e. in proximity of the lesion 94. With the guiding catheter 10 (catheter body 11) secured, endoscopic probe 20 can be navigated beyond the outlet aperture 122 of catheter 10 for visual inspection of the region of the lesion 95. An image 99 of the lesion can be taken by the endoscopic probe 20 and possibly visualized on visual display 42 for inspection.

If the endoscopic probe is slightly taken inside the internal lumen 12, one can take advantage of the porthole effect in order to improve vision by the imaging device 22. By way of example, the distal tip or outlet aperture 122 of the catheter body can be held against the anatomical target structure, while (proximally) retracting the endoscopic probe 20 inside the internal lumen to suitably image the anatomical structure by the porthole effect. By so doing, the distal tip of the catheter body advantageously maintains the anatomical structure in a desired position.

A lens or screen of the imaging device 22 may get soiled or smudged by body fluids while navigating, impairing proper visualization. Fluid is supplied via the second inlet port 322 of fluid delivery module 30 (e.g. by operating valve 351) to the internal lumen 12 which flushes the tip of the endoscopic probe 20 and cleans it. Flushing can be performed when the distal portion 23 is completely received inside the internal lumen 12, or the (external) lens of imaging device 22 is flush with the outlet aperture 122, or at least part of the distal portion 23 still extending distally beyond the outlet aperture 122. The imaging device 22 is then ready to be deployed again at or beyond the outlet aperture 122 of the guiding catheter 10.

At operation 88, a diagnostic or surgical procedure is executed. A measurement of an anatomical structure, e.g. a diameter or length, can be taken, possibly with the aid of endoscopic probe 20. By way of example, a measurement can be taken on the basis of a visual image taken by the imaging device 22. It is alternatively possible to determine a length within an anatomical structure by advancing or retracting the endoscopic probe 20 relative to the guiding catheter 10. The imaging device 22 can hereby serve as a visual aid to determine beginning and end of the anatomical structure of interest. The control unit 40 can be configured to determine a measurement from one or more images taken by the imaging device 22. By way of example, control unit 40 can comprise a user interface allowing an operator to select positions on the visual display 42 and the control unit implemented with a computing unit configured to determine a measurement based on the selected positions.

Alternatively, or in addition, the endoscopic probe 20 can be removed from guiding catheter 10 and another surgical instrument deployed through the internal lumen 12 instead, such as a biopsy forceps or brush. Yet alternatively, the surgical instrument may be deployed through another instrument channel (not shown) of endoscope 70 to arrive at the anatomical target region. Here as well, the endoscopic probe can be utilised as a visual aid for navigating and/or operating the surgical instrument.

It will be appreciated that some of the operations 81 through 88 can be omitted. Particularly, operations 81, 82, 84 and 88 are optional. In addition, the operations 81-88 need not be executed in the order as described or depicted in the diagram of Fig. 11, but can be executed in any appropriate order.

Assemblies of the present disclosure are particularly suited for surgical and/or diagnostic operations in the pulmonary tract, or the bilio-pancreatic tract, e.g. the biliary duct.

Some aspects of the present disclosure are set out in the following alphanumerically ordered clauses.
A1. A method of performing endoscopy, comprising:
   navigating an endoscope device, preferably a bronchoscope, through an anatomical cavity or lumen, particularly until arriving at an anatomical park position;
   deploying the guiding catheter according to aspects of the present disclosure through an instrument channel of the endoscope device, preferably beyond the distal tip of the endoscope device, preferably to arrive at an anatomical target location;
   securing a position of the guiding catheter with respect to a reference;
   deploying the endoscopic probe according to aspects of the present disclosure through the guiding catheter and imaging the anatomical target location by the imaging device of the endoscopic probe.
A2. The method of clause A1, further comprising determining a navigation route along the anatomical cavity or lumen, wherein navigating the endoscope device comprises navigating the endoscope device along the navigation route, preferably wherein the navigation route comprises the anatomical park position and/or the anatomical target location.
A3. The method of clause A2, further comprising tracking a trackable marker along the navigation route.
A4. The method of clause A3, comprising deploying a tracking probe comprising the trackable marker through an instrument channel of the endoscope device.
A5. The method of clause A4, comprising retracting the tracking probe following securing the position the guiding catheter.
A6. The method of clause A5, wherein the endoscopic probe is deployed through the guiding catheter following retracting the tracking probe.
A7. The method of clause A4, wherein the tacking probe is the endoscopic probe.
A8. The method of any one of the clauses A3 to A7, wherein determining the navigation route comprises determining a virtual or digital model of the anatomical cavity or lumen and mapping the virtual or digital model to an image of the anatomical cavity or lumen captured by the endoscope device and/or the endoscopic probe, wherein a position of the image is determined based on tracking the trackable marker.

## Claims

1. Assembly (100, 200) for endoluminal navigation, comprising:
a guiding catheter (10) having a proximal end (101) and a distal end (102), wherein the guiding catheter comprises an elongate flexible tubular catheter body (11) defining an internal lumen (12) of the catheter, wherein the internal lumen (12) comprises an outlet aperture (122) at the distal end (102),
an endoscopic probe (20), comprising a flexible probe body (21) and an imaging device (22) attached to the probe body at a distal end of the probe body, wherein the probe body and the imaging device are sized to be slidingly received in the internal lumen (12), and
a fluid delivery module (30) attached or attachable to the guiding catheter (10) at the proximal end (101), wherein the fluid delivery module comprises a first port (321) configured to communicate with the internal lumen (12), wherein the first port is configured to receive the endoscopic probe (20) for deployment through the internal lumen, and a second port (322) configured to deliver a fluid to the internal lumen (12),
wherein the first port (321) comprises a fluid seal (34) configured to fluid tightly seal the first inlet port (321) against the probe body (21) while the second port (322) remains in fluid communication with the internal lumen (12).

2. Assembly of claim 1, wherein the fluid seal (34) comprises a seal member (341) provided with a through-bore configured to slidingly receive the probe body (21), preferably wherein the fluid seal comprises a tightening means (342, 343) configured to tighten the sealing member (341) against the probe body (21).

3. Assembly of claim 1 or 2, further comprising a fluid source (35) configured to be coupled to the second port (322).

4. Assembly of claim 3, further comprising a flow control valve (351) coupled between the second port (322) and the fluid source (35).

5. Assembly (200) of any one of the preceding claims, further comprising an attachment module (50), wherein the attachment module comprises a connector system (54) and a telescopic tubular body (57), the telescopic tubular body extending from a first end (52) to a second end (51) opposite the first end and defining an internal channel (56) of the attachment module (50), wherein the internal channel is sized to slidingly receive the catheter body (11), wherein the connector system (54) is provided at the second end (51) and is configured to secure the attachment module (50) to a support (60).

6. Assembly of claim 5, wherein the attachment module further comprises a handle member (58) attached to the telescopic tubular body (57) at the first end (52) and a first locking system (59) configured to lock a motion of the guiding catheter (10) relative to the handle member in respect of at least one of: a translation along a longitudinal axis (209) of the telescopic tubular body and a rotation about the longitudinal axis.

7. Assembly of claim 6, wherein the guiding catheter (10) comprises a first connector member (131) configured to releasably interlock with a corresponding connector member (591) of the locking system (59) so as to enable an integral motion of the guiding catheter and the handle member.

8. Assembly of any one of claims 5 to 7, wherein the attachment module (50) further comprises a second locking system (55) configured to fixedly secure the guiding catheter (10) axially relative to the support (60), preferably wherein the second locking system (55) is arranged at the second end (51).

9. Assembly of any one of the preceding claims, wherein the catheter body (11) is configured to transmit a torque and/or a rotation from the proximal end (101) to the distal end (102) so as to navigate the guiding catheter (10).

10. Assembly of any one of the preceding claims, wherein the endoscopic probe (20) comprises a distal portion (23) at the distal end of the probe body (21), wherein the distal portion accommodates the imaging device (22) and has an increased diameter with respect to a diameter of the probe body (21).

11. Assembly of claim 10, wherein a ratio of a diameter of the probe body (21) to a diameter of the internal lumen (12) is between 0.15 and 0.9 and a ratio of a diameter (D₁) of the distal portion (23) to the diameter of the internal lumen (12) is between 0.3 and 0.95.

12. Assembly of any one of the preceding claims, wherein the imaging device (22) comprises a camera module (221) and a light source (222).

13. Assembly of any one of the preceding claims, further comprising an endoscope (70), wherein the endoscope comprises an endoscope handle (60) connected to an endoscope body (75), the endoscope body comprising an instrument channel (71), wherein the endoscope handle comprises an entry port (61) communicating with the instrument channel (71), wherein the guiding catheter (10) is configured to be slidingly received in the instrument channel (71) and through the entry port (61).

14. Assembly of claim 13, wherein the endoscope (70) further comprises a second imaging device (73) arranged at a distal end of the endoscope body (75).

15. Assembly of claim 13 or 14, further comprising the attachment module (50) as recited in any one of the claims 5 to 8, wherein the endoscope handle (60) forms the support and comprises a connector part (62), wherein the connector system (54) is configured to co-operate with the connector part to attach the attachment module (50) to the endoscope handle (60).
